# FASCICULE DE BREVET EUROPEEN

(11) **EP 2 173 555 B1**
(45) Date de publication et mention de la délivrance du brevet: **04.09.2013**
(21) Numéro de dépôt: 08826958.4
(22) Date de dépôt: 10.07.2008
(51) Int. Cl.: B32B 27/18, B32B 27/32, A61K 31/34, A61K 9/14, B65D 81/24

(54) **UTILISATION D'UN MATERIAU A BASE DE POLYMERE THERMOPLASTIQUE A FORTE TENEUR EN AGENTS ANTIOXYDANTS POUR L'EMBALLAGE DE DIANHYDROHEXITOLS**
VERWENDUNG EINES MATERIALS AUF BASIS EINES THERMOPLASTISCHEN POLYMERS MIT HOHEM ANTEIL VON ANTIOXIDANTIEN ZUR VERPACKUNG VON DIANHYDROHEXITOLEN
USE OF A MATERIAL BASED ON A THERMOPLASTIC POLYMER HAVING A HIGH CONTENT OF ANTIOXIDANTS FOR PACKAGING DIANHYDROHEXITOLS

(30) Priorité: 02.08.2007 FR 0705653
(43) Date de publication de la demande: 14.04.2010
(73) Titulaire: Roquette Freres, 62136 Lestrem (FR)
(72) Inventeur: FUERTES, Patrick, F-59160 Lomme (FR); INGRET, Maxime, F-62400 Bethune (FR); LAMBIN, Anne, F-59160 Lomme (FR); WYART, Hervé, F-62149 Cuinchy (FR)
(74) Mandataire: Cabinet Plasseraud
(86) Numéro de dépôt international: PCT/FR2008/051296
(87) Numéro de publication internationale: WO 2009/019371

(56) Documents cités:
- EP-B- 1 287 000
- WO-A-03/043959
- DATABASE WPI Week 200634 Thomson Scientific, London, GB; AN 2006-324989 XP002473828 & JP 2006 117649 A (NIKKEN CHEM CO LTD) 11 mai 2006 (2006-05-11) & JP 2006 117649 A (NIKKEN CHEMICALS CO LTD) 11 mai 2006 (2006-05-11) cité dans la demande

## Description

La présente invention concerne l'utilisation d'un matériau thermoplastique particulier contenant une couche présentant une teneur importante en agents antioxydants, pour emballer des dianhydrohexitols.

Les dianhydrohexitols, également appelés isohexides, sont des produits de déshydratation interne de sucres hydrogénés en C₆ (hexitols) tels que le sorbitol, le mannitol et l'iditol.

Parmi ces sucres hydrogénés doublement déshydratés, l'isosorbide est aujourd'hui celui pour lequel on développe et envisage de développer le plus d'applications industrielles, notamment dans le domaine pharmaceutique, dans le domaine des intermédiaires de synthèse chimique et dans le domaine des matières plastiques.

Pour la majorité de ces applications, il est généralement nécessaire de disposer de compositions les plus pures possible, ayant notamment une teneur en dianhydrohexitol au moins égale à 98,5 % en poids, de préférence au moins égale à 99,5 % % en poids.

La constatation que les dianhydrohexitols, et en particulier l'isosorbide, étaient des produits non seulement fortement hygroscopiques mais chimiquement peu stables est relativement récente.

La Demanderesse a en particulier observé que le stockage d'isosorbide fabriqué selon des procédés connus, même à l'abri de l'humidité de l'atmosphère, pouvait entraîner dans certaines conditions de température une dégradation chimique aboutissant, entre autres, à la formation d'acide formique, acide qui présente une odeur caractéristique et désagréable, particulièrement gênante dans des applications pharmaceutiques ou autres.

La Demanderesse a ainsi été amenée à mettre au point des procédés de purification et de stabilisation de dianhydrohexitols décrits notamment dans les demandes de brevets EP 1 287 000 et WO 03/043959.

Dans le cadre des recherches ayant abouti aux inventions divulguées dans ces demandes, la stabilité des produits a été évaluée par stockage des échantillons à tester dans un récipient, respectivement en plastique (EP 1 287 000) ou en verre (WO 03/043959), à une température thermostatée égale à, respectivement, 60°C ou 40°C. Ces essais de stabilité avaient ainsi permis de prévoir les stabilités au stockage des produits commercialisés par la Demanderesse.

Ce n'est que très récemment que la Demanderesse s'est aperçue du fait que les durées de conservation des dianhydrohexitols, déterminées dans les conditions des tests de stabilité décrits dans les demandes EP 1 287 000 et WO 03/043959, ne reflétaient qu'imparfaitement la stabilité de ces mêmes produits dans les conditions réelles de transport et de stockage. La Demanderesse a relevé en particulier dans certains cas des concentrations d'acide formique relativement plus importantes à proximité du film d'emballage en polyéthylène. Cette concentration localement élevée pouvait laisser penser que la dégradation de l'isosorbide, et des dianhydrohexitols en général, ne se déroulait pas seulement selon une cinétique intrinsèque, dépendante de la température, mais était aussi liée, entre autres, à l'interaction avec le matériau d'emballage.

Cette découverte était pour le moins surprenante. En effet, le polyéthylène, matière plastique universellement utilisée pour l'emballage de produits techniques, pharmaceutiques et alimentaires, a la réputation d'être un matériau stable, inerte et inoffensif. Son remplacement par une matière plastique différente pose un certain nombre de problèmes : en effet, le polyéthylène fait partie des polymères les moins chers du marché et présente, entre autres, une excellente aptitude au thermosoudage. Cette dernière caractéristique peut s'avérer essentielle pour l'emballage de produits hygroscopiques et/ou chimiquement instables tels que les dianhydrohexitols, qui sont de préférence conservés dans des emballages hermétiques, imperméables à l'oxygène et à la vapeur d'eau.

La Demanderesse s'est donc fixé pour but de trouver un matériau plastique peu coûteux et thermosoudable, permettant d'emballer des compositions de dianhydrohexitols à pureté élevée, et qui, contrairement au polyéthylène classique, n'accélère pas la dégradation chimique des dianhydrohexitols.

La demande de brevet JP 2006-117649 divulgue l'utilisation d'un matériau d'emballage de type film pour emballer de l'isosorbide dans le but de préserver celui-ci contre l'absorption d'eau, de le maintenir sous forme de poudre fluide et d'empêcher la formation d'agrégats. Le film d'emballage est défini très vaguement comme étant un film multicouches à base de matières plastiques et d'aluminium. Ce document ne mentionne pas, en outre, le problème de l'instabilité chimique des dianhydrohexitols au contact du matériau d'emballage.

Dans le cadre de ses recherches visant à mettre au point un tel matériau d'emballage ou à le sélectionner parmi les matériaux d'emballage connus, la Demanderesse a constaté avec surprise qu'il était possible d'améliorer considérablement la stabilité des dianhydrohexitols, et en particulier de l'isosorbide, en augmentant de manière substantielle la concentration en agents antioxydants de la couche de matière plastique en contact avec le produit. Elle a en outre fait la découverte, encore plus surprenante, que l'incorporation d'une telle quantité d'agent antioxydant avait un effet stabilisant non seulement lorsque l'agent antioxydant se trouvait dans la couche directement en contact avec le produit, mais également lorsqu'il était introduit dans la couche immédiatement adjacente à la couche en contact avec le produit, à condition que cette dernière ne présente pas une épaisseur excessive.

La présente invention a par conséquent pour objet un dianhydrohexitol emballé dans un matériau d'emballage à base de polymère thermoplastique, caractérisé par le fait que le matériau d'emballage comprend au moins une couche de polymère thermoplastique contenant au moins 0,1 % en poids, de préférence au moins 0,2 % en poids, d'au moins un agent antioxydant (couche (A)), ladite couche (A) étant soit directement en contact avec le dianhydrohexitol soit étant séparée de celui-ci par une couche supplémentaire en polymère thermoplastique (couche (B)) ayant une épaisseur au plus égale à 150 µm, de préférence au plus égale à 120 µm, et par le fait que le polymère thermoplastique de la couche (A) et/ou de la couche (B) est choisi indépendamment parmi le polyéthylène, le polypropylène, les copolymères d'éthylène et de propylène, et les mélanges de ceux-ci.

L'invention a en outre pour objet l'utilisation d'un tel matériau d'emballage à base de polymère thermoplastique pour emballer un dianhydrohexitol, ou encore un procédé d'emballage d'un dianhydrohexitol, comprenant l'introduction dudit dianhydrohexitol dans un emballage en un tel matériau, et la fermeture dudit emballage.

Les dianhydrohexitols (1,4 - 3,6-dianhydro-hexitols) englobent l'isosorbide (1,4 - 3,6-dianhydro-sorbitol), l'isomannide (1,4 - 3,6 dianhydro-mannitol), l'isoidide (1,4 - 3,6-dianhydro-iditol) et les mélanges d'au moins deux de ces produits. De préférence, le dianhydrohexitol emballé selon la présente invention comprend de l'isosorbide ou est essentiellement constitué d'isosorbide. Il s'agit de préférence d'une composition dont la teneur en isosorbide est au moins égale à 98,5 % en poids (sec/sec).

Bien que l'utilité de la présente invention s'applique en principe aux compositions tant solides que liquides de dianhydrohexitols, elle est particulièrement importante pour les formes solides.

En tant que formes solides, il peut s'agir, par exemple, de distillats refroidis et solidifiés ou de cristaux, l'ensemble de ces produits pouvant notamment se présenter sous forme d'une poudre ou d'écailles.

L'utilisation d'un matériau plastique tel que défini dans la présente demande pour l'emballage de dianhydrohexitols sous forme solide, et notamment sous forme cristalline anhydre, constitue par conséquent un mode de réalisation préféré de la présente invention.

Le terme « agent anti-oxydant » utilisé dans la présente invention englobe tous les composés capables de limiter ou de supprimer la dégradation thermo-oxydative, également connue sous le terme d'auto-oxydation, de composés organiques, en particulier de polymères organiques.

Une liste non exhaustive de ces composés est donnée dans le chapitre 1, intitulé « Antioxidants », de la 5ème édition de l'ouvrage « Plastics Additives Handbook » (2001), Carl Hanser Verlag, Münich (Allemagne).

Parmi les composés anti-oxydants préférés, on peut citer,
- les donneurs d'hydrogène tels que les amines aromatiques secondaires et les phénols à encombrement stérique important,
- les agents de décomposition des hydroperoxydes à base de phosphore tels que les phosphites et phosphonites, et ceux à base de soufre tels que les esters d'acide 3,3-thiodipropionique, et
- les agents piégeurs de radicaux libres tels que le noir de carbone, les amines à fort encombrement stérique, les hydroxylamines, les dérivés de benzofuranone et les phénols modifiés par des groupes acryloyle.

Les donneurs d'hydrogène de type phénols englobent par exemple ceux portant les numéros CAS suivants : 10191-41-0 (tocophérol), 128-37-0, 2082-79-3, 12643-61-0, 119-47-1, 35074-77-2, 23128-74-7, 976-56-7, 65140-91-2, 36443-68-2, 85-60-9, 90498-90-1, 1709-70-2, 1843-03-4, 34137-09-2, 27676-62-6, 40601-76-1, 6683-19-8, 32509-66-3, 31851-03-3, 134701-20-5-, 96-69-5, 90-66-4, 110553-27-0, 41484-35-9, 991-84-4, 103-99-1, 63843-89-0, 4221-80-1, 67845-93-6, 136-36-7, 61167-58-6, 128961-68-2, 181314-48-7, 143925-92-2, 135-88-6, 26780-96-1, 101-72-4, 90-30-2, 68411-46-1, 10081-67-1 et 118832-72-7.

Les agents de décomposition des hydroperoxydes de type phosphite ou phosphonite englobent par exemple ceux portant les numéros CAS suivants : 26523-78-4, 31570-04-4, 26741-53-7, 80693-00-1, 140221-14-3, 119345-01-6/38613-77-3, 118337-09-0, 3806-34-6, 80410-33-9, 145650-60-8, 161717-32-4 et 154862-43-8. Les agents de décomposition des hydroperoxydes à base de soufre englobent les composés portant les numéros CAS suivants : 693-36-7, 123-28-4, 16545-54-3 et 2500-88-1.

Les amines à fort encombrement stérique englobent les composés portant les numéros CAS suivants : 52829-07-9, 65447-77-0, 71878-19-8, 106990-43-6, 41556-26-7, 63843-89-0, 129757-67-1, 192268-64-7, 90751-07-8, 219920-30-6, 79720-19-7, 106917-30-0, 24860-22-8, 131290-28-3, 109-423-00-9, 124172-53-8, 199-237-39-3, 91788-83-9, 64022-61-3, 107119-91-5, 100631-43-4, 115055-30-6, 100631-44-5, 95078-42-5, 85099-51-1/85099-50-9, 78276-66-1, 76505-58-3, 136504-96-6, 71029-16-8, 96204-36-3, 130277-45-1, 85099-51-0, 147783-69-5, 154636-12-1, 84214-94-8, 99473-08-2, 164648-93-5 et 42774-15-2.

Dans un mode de réalisation particulièrement préféré de la présente invention, l'agent anti-oxydant comprend du noir de carbone ou est essentiellement constitué de noir de carbone. Ce noir de carbone doit avantageusement avoir une granulométrie suffisamment fine pour pouvoir être incorporé de façon homogène dans la couche (A). La granulométrie la plus appropriée du noir de carbone dépend donc notamment de l'épaisseur de la couche (A).

Qu'il s'agisse de noir de carbone ou d'un autre agent anti-oxydant choisi parmi ceux indiqués dans le Plastics Additives Handbook référencé ci-dessus, la concentration de l'agent anti-oxydant est de préférence comprise entre 0,1 et 10 % en poids, en particulier entre 0,2 et 5 % en poids et encore plus préférentiellement entre 0,3 et 3 % en poids.

Comme indiqué ci-dessus, la couche (A) contenant l'agent anti-oxydant peut être directement en contact avec le dianhydrohexitol. Dans ce mode de réalisation, il est particulièrement préconisé de veiller à ce que la granulométrie dudit agent, en particulier du noir de carbone, soit relativement faible par rapport à l'épaisseur de la couche (A). Il est en effet particulièrement avantageux que les particules dudit agent, en particulier du noir de carbone, soient parfaitement incorporées dans la couche de polyéthylène, de polypropylène et/ou de copolymères d'éthylène et de propylène, de manière à ce que la surface interne de l'emballage soit lisse et ne comporte pas de particules saillantes, susceptibles de se décoller ou de se briser et de polluer ainsi le produit emballé.

Dans un deuxième mode de réalisation, la couche (A) contenant l'agent anti-oxydant est séparée du dianhydrohexitol par une couche (B) en polymère thermoplastique, exempte d'agent antioxydant ou ayant une teneur en agent anti-oxydant inférieure à 0,1 % en poids, de préférence inférieure à 0,05 % en poids.

Cette couche (B) supplémentaire, intercalée entre le dianhydrohexitol et la couche (A) chargée en agent anti-oxydant, est de préférence essentiellement constituée de polyéthylène (PE), de polypropylène (PP), de copolymères d'éthylène et de propylène (PE/PP) ou de mélanges de ceux-ci.

Selon une variante avantageuse, la couche (B) est une couche en polyéthylène non chargé avec du noir de carbone et qui est directement en contact avec le dianhydrohexitol.

Cette couche (B) peut toutefois être une structure de type multicouches, constituée elle-même de deux couches ou plus, dont une ou plusieurs sont à base de PE, PP, PE/PP ou d'un mélange de ceux-ci, et éventuellement une ou plusieurs autres à base d'un polymère thermoplastique différent.

Bien entendu, les indications concernant l'épaisseur de la couche (B) de l'emballage utilisé dans la présente invention concernent soit l'épaisseur de la structure monocouche, soit, le cas échéant, l'épaisseur totale de l'ensemble des couches formant la structure multicouches.

Comme indiqué en introduction, cette épaisseur de la couche (B), qu'elle soit mono- ou multicouche, ne doit pas dépasser 150 µm. Au-delà de cette valeur maximale, l'effet stabilisant de l'agent anti-oxydant présent dans la couche (A) adjacente devient en effet insuffisant. La couche (B) a de préférence une épaisseur au plus égale à 120 µm, avantageusement comprise entre 10 et 100 µm, et en particulier entre 15 et 50 µm.

Comme indiqué ci-dessus, le matériau d'emballage de la présente invention comprend au moins une couche à base de PE, PP, PE/PP ou d'un mélange de ceux-ci. Ces polymères ou associations de polymères sont choisis en raison de leur excellente aptitude au thermosoudage, de leur faible coût et de leur grande disponibilité.

Le polymère thermoplastique de la couche (A) et/ou de la couche (B) est de préférence du polyéthylène. Il s'agit en particulier de polyéthylène ramifié, obtenu par polymérisation radicalaire, de polyéthylène linéaire, préparé par polymérisation Ziegler-Natta et/ou de polyéthylène métallocène, obtenu par polymérisation par catalyse zinc/zirconium.

Selon un mode de réalisation préféré de la présente invention, la couche (A) est en polyéthylène chargé avec du noir de carbone et la couche (B) est en polyéthylène non chargé en noir de carbone et est directement en contact avec le dianhydrohexitol.

Dans un autre mode de réalisation préféré, le dianhydrohexitol est emballé hermétiquement, c'est-à-dire que l'emballage, par exemple le sachet, la sache ou le sac, contenant le dianhydrohexitol est fermé, par exemple par thermosoudage ou au moyen d'un lien approprié, de manière à limiter au maximum, et si possible de manière à supprimer, tout échange de gaz entre l'intérieur de l'emballage et l'atmosphère ambiante. L'étanchéité de l'emballage est particulièrement importante pour l'isosorbide qui, à l'état solide, est un produit extrêmement hygroscopique.

Dans certaines conditions, il peut être intéressant d'emballer le dianhydrohexitol sous atmosphère anhydre et/ou inerte, par exemple sous atmosphère d'azote.

Pour garantir une stabilité optimale du dianhydrohexitol au cours du transport et du stockage il peut parfois être utile, voire nécessaire, de prévoir une couche supplémentaire de protection, partielle ou totale, contre l'oxygène de l'air, la vapeur d'eau et/ou la lumière. Une telle couche barrière peu perméable, voire imperméable, à la vapeur d'eau et à l'oxygène (couche (C)), et de préférence également à la lumière, est de préférence située à l'extérieur de la couche (A), c'est-à-dire qu'elle n'est pas intercalée entre la couche (A) et le produit emballé. Cette couche (C) est de préférence en contact direct avec la surface extérieure de la couche (A).

On peut citer à titre d'exemples de telles couches barrière (C) des couches à base de copolymère éthylène/alcool vinylique (« EVOH »), de poly(chlorure de vinylidène) (« PVDC »), de polyamide (« PA »), de polyacrylonitrile (« PAN ») et/ou de poly(acide glycolique) (« PGA »). La couche barrière (C) peut également être un dépôt d'aluminium et/ou d'un autre métal approprié, déposé par exemple sur la surface extérieure de la couche (A) ou une feuille d'aluminium et/ou d'un autre métal approprié, de préférence en contact direct, avec la surface extérieure de la couche (A).

L'épaisseur globale de l'emballage ne joue pas un rôle déterminant dans la présente invention. Il peut en effet s'agir d'un matériau mince et souple, par exemple de type film ou feuille, dont l'épaisseur ne dépasse pas quelques dizaines ou quelques centaines de microns, mais également d'un matériau plus rigide sous forme de récipient ayant une forme déterminée. Pour des raisons de coût essentiellement, le matériau d'emballage présente de préférence une épaisseur totale au plus égale à 300 µm. Un matériau d'emballage selon l'inventiom ayant une épaisseur totale comprise entre 30 et 250 µm permet généralement d'atteindre un compromis satisfaisant entre solidité mécanique suffisante et coût de production. L'emballage peut alors se présenter avantageusement sous la forme de sachets, saches ou sacs de toutes formes, dimensions et contenances et par exemple d'une sache (en anglais « liner » ou « pouch ») qui, en vue du transport ou du stockage du dianhydrohexitol, peut être déjà contenue ou être destinée à être contenue dans un récipient souple tel qu'un sac en aluminium ou un « big-bag » (ou « Grand Récipient Vrac Souple » = GRVS ou « Flexible Intermediate Container » = FIS) en toile ou tissu, ou dans un récipient rigide tel qu'une caisse en carton.

La Demanderesse a obtenu en particulier d'excellents résultats, en termes de stabilité au stockage, avec un film d'une épaisseur totale inférieure à 150 µm, comprenant une couche (A) en polyéthylène chargé avec 2% en poids environ de noir de carbone et une couche (B) en polyéthylène contenant moins de 0,05 % en poids total d'agents antioxydants et en particulier exempte de noir de carbone, directement en contact avec le dianhydrohexitol. Grâce au revêtement interne en polyéthylène (couche (B)), le film est facile à souder avec les machines classiques de thermosoudage et il n'y a aucun risque de contamination du produit emballé par le noir de carbone.

La supériorité d'un tel film d'emballage sur quatre films classiques en polyéthylène, contenant moins de 0,05% en poids total d'agents antioxydants et en particulier exempts de noir de carbone, est illustrée dans l'exemple ci-après qui a un caractère purement illustratif.

### Exemple 1

50 g d'isosorbide sous forme solide (écailles) sont introduits dans un sachet (25 cm x 25 cm) constitué du matériau d'emballage à tester. Le sachet est immédiatement fermé par soudage à l'aide d'une soudeuse thermique à impulsions (modèle SZ 380 commercialisé par la société Joisten & Kettenbaum GmbH & Co, Bergisch Gladbach, Allemagne). Le sachet ainsi scellé est à son tour introduit dans un deuxième sachet en aluminium comportant un revêtement en polyéthylène, fermé par soudage à l'aide de la même thermosoudeuse afin d'assurer l'étanchéité vis-à-vis de l'atmosphère extérieure. Les échantillons ainsi emballés sont placés dans une étuve ventilée, thermostatée à une température de 50 °C. Un échantillon témoin est enfermé dans un flacon en verre et stocké dans les mêmes conditions.

Après une période déterminée, la totalité de l'échantillon d'isosorbide est extrait des emballages et dissous à 40 % en poids de matière sèche dans de l'eau osmosée. On mesure pour chaque échantillon le pH de la solution.

Les résultats de pH-métrie sont présentés dans le tableau 1 ci-après.

**Tableau 1**

| Stabilité au stockage de l'isosorbide dans différents films d'emballage à base de polyéthylène | | | | | |
|---|---|---|---|---|---|
| Durée de stockage | PE + Noir de carbone (selon l'invention) | Sachet 1 (comparatif) **R* / L*** (50%/50 %) | Sachet 2 (comparatif) **R* / L*** (70%/30 %) | Sachet 3 (comparatif) 100 % de **R*** | Sachet 4 (comparatif) 100 % de L* |
| 0 jour | pH 7,6 | pH 7,6 | pH 7,6 | pH 7,6 | pH 7,6 |
| 2 semaines | pH 7,2 | pH 7,5 | pH 7,5 | **pH 3,6** | pH 7,2 |
| 1 mois | pH 7,3 | pH 7,4 | **pH 3,1** | | **pH 2,9** |
| 1,5 mois | pH 7,4 | pH 7,4 | | | |
| 2 mois | pH 7,5 | **pH 3,0** | | | |
| 2,5 mois | pH 7,5 | | | | |
| 3 mois | pH 7,6 | | | | |
| 4 mois | pH 7,7 | | | | |

| | | | | | |
|---|---|---|---|---|---|
| R* = polyéthylène ramifié ; L* = polyéthylène linéaire | | | | | |

Le pH de l'échantillon témoin, conservé dans un récipient en verre, reste globalement stable (pH variant entre 7,2 et 7,7) jusqu'à 3 mois. Après 4 mois de conservation, on mesure toutefois une baisse très significative du pH qui chute à une valeur de 3,0.

Ces exemples montrent clairement la supériorité du film d'emballage utilisable selon l'invention par rapport aux quatre films de polyéthylène classiques, contenant de faibles teneurs en agents antioxydants et exempts de noir de carbone, et même par rapport à l'emballage en verre.

L'ensemble des échantillons non emballés conformément à l'invention présentaient après 4 mois (emballage en verre), voire après seulement 2 mois (sachet 1), 1 mois (sachets 2 et 4) ou même 2 semaines (sachet 3), un pH significativement inférieur à 4 et une odeur caractéristique d'acide formique.

A l'inverse, l'isosorbide emballé conformément à l'invention ne présentait aucune baisse de pH après 4 mois de stockage. Une analyse faite à ce moment là sur le produit emballé ne montrait aucune présence de peroxydes, c'est-à-dire de marqueurs de phénomènes d'oxydation et donc d'instabilité.

## Revendications

1. Dianhydrohexitol, de préférence sous forme solide, emballé dans un matériau d'emballage à base de polymère thermoplastique, **caractérisé par le fait que** le matériau d'emballage comprend au moins une couche de polymère thermoplastique contenant au moins 0,1 % en poids, de préférence au moins 0,2 % en poids, d'au moins un agent anti-oxydant (couche (A)), ladite couche (A) étant soit directement en contact avec le dianhydrohexitol soit étant séparée de celui-ci par une couche en polymère thermoplastique (couche (B)) ayant une épaisseur au plus égale à 150 µm, de préférence au plus égale à 120 µm, et **par le fait que** le polymère thermoplastique de la couche (A) et/ou de la couche (B) est choisi indépendamment parmi le polyéthylène, le polypropylène, les copolymères d'éthylène et de propylène, et les mélanges de ceux-ci.

2. Dianhydrohexitol emballé selon la revendication 1, **caractérisé par le fait que** la couche (B) a une épaisseur comprise entre 10 et 100 µm, de préférence comprise entre 15 et 50 µm.

3. Dianhydrohexitol emballé selon la revendication 1 ou 2, **caractérisé par le fait que** le dianhydrohexitol comprend de l'isosorbide ou est essentiellement constitué d'isosorbide.

4. Dianhydrohexitol emballé selon l'une quelconque des revendications précédentes, **caractérisé par le fait que** le polymère thermoplastique de la couche (A) et/ou de la couche (B) est du polyéthylène, de préférence du polyéthylène ramifié, du polyéthylène linéaire et/ou du polyéthylène métallocène.

5. Dianhydrohexitol emballé selon l'une quelconque des revendications précédentes, **caractérisé par le fait que** le dianhydrohexitol est emballé hermétiquement, de préférence sous atmosphère inerte.

6. Dianhydrohexitol emballé selon l'une quelconque des revendications précédentes, **caractérisé par le fait que** l'agent anti-oxydant est du noir de carbone.

7. Dianhydrohexitol emballé selon l'une quelconque des revendications précédentes, **caractérisé par le fait que** le matériau d'emballage comprend une couche (A) en polyéthylène chargé avec du noir de carbone et une couche (B) en polyéthylène non chargé avec du noir de carbone, directement en contact avec le dianhydrohexitol.

8. Dianhydrohexitol emballé selon l'une quelconque des revendications précédentes, **caractérisé par le fait que** le matériau d'emballage comprend en outre une couche barrière peu perméable ou imperméable à la vapeur d'eau et à l'oxygène (couche (C)), située à l'extérieur de la couche (A).

9. Dianhydrohexitol emballé selon l'une quelconque des revendications précédentes, **caractérisé par le fait que** le matériau d'emballage à base de polymère thermoplastique a une épaisseur totale au plus égale à 300 µm, de préférence comprise entre 30 et 250 µm.

10. Dianhydrohexitol emballé selon l'une quelconque des revendications précédentes, **caractérisé par le fait que** la couche (A) contient de 0,1 à 10 % en poids, de préférence de 0,2 à 5 % en poids et en particulier de 0,3 à 3 % en poids d'agent anti-oxydant.

11. Utilisation d'un matériau d'emballage à base de polymère thermoplastique pour emballer un dianhydrohexitol, de préférence sous forme solide, **caractérisé par le fait que** le matériau d'emballage comprend au moins une couche de polymère thermoplastique contenant au moins 0,1 % en poids, de préférence au moins 0,2 % en poids, d'au moins un agent anti-oxydant (couche (A)), ladite couche (A) étant soit directement en contact avec le dianhydrohexitol soit étant séparée de celui-ci par une couche en polymère thermoplastique (couche (B)) ayant une épaisseur au plus égale à 150 µm, de préférence au plus égale à 120 µm, et **par le fait que** le polymère thermoplastique de la couche (A) et/ou de la couche (B) est choisi indépendamment parmi le polyéthylène, le polypropylène, les copolymères d'éthylène et de propylène, et les mélanges de ceux-ci.

12. Procédé d'emballage de dianhydrohexitol, de préférence sous forme solide, comprenant l'introduction dudit dianhydrohexitol dans un emballage en un matériau à base de polymère thermoplastique, et la fermeture dudit emballage, ledit procédé étant **caractérisé par le fait que** le matériau d'emballage comprend au moins une couche de polymère thermoplastique contenant au moins 0,1 % en poids, de préférence au moins 0,2 % en poids, d'au moins un agent anti-oxydant (couche (A)), ladite couche (A) étant soit directement en contact avec le dianhydrohexitol soit étant séparée de celui-ci par une couche en polymère thermoplastique (couche (B)) ayant une épaisseur au plus égale à 150 µm, de préférence au plus égale à 120 µm, et **par le fait que** le polymère thermoplastique de la couche (A) et/ou de la couche (B) est choisi indépendamment parmi le polyéthylène, le polypropylène, les copolymères d'éthylène et de propylène, et les mélanges de ceux-ci.

## Patentansprüche

1. Dianhydrohexitol, vorzugsweise in fester Form, verpackt in einem Verpackungsmaterial auf Basis eines thermoplastischen Polymers, **gekennzeichnet dadurch, dass** das Verpackungsmaterial mindestens eine Schicht aus thermoplastischem Polymer umfasst, enthaltend mindestens 0,1 Gew.-%, vorzugsweise mindestens 0,2 Gew.-%, mindestens eines Antioxidationsmittels (Schicht (A)), wobei die Schicht (A) entweder direkt in Kontakt ist mit dem Dianhdrohexitol oder getrennt ist von diesem durch ein thermoplastisches Polymer (Schicht (B)) das eine Dicke von höchstens gleich 150 µm, vorzugsweise von höchstens gleich 120 µm aufweist, und dadurch, dass das thermoplastische Polymer der Schicht (A) und/oder der Schicht (B) unabhängig ausgewählt ist aus Polyethylen, Polypropylen, den Copolymeren von Ethylen und Propylen und den Gemischen von diesen.

2. Dianhydrohexitol, verpackt nach Anspruch 1, **dadurch gekennzeichnet, dass** die Schicht (B) eine Dicke aufweist, die zwischen 10 und 100 µm, vorzugsweise zwischen 15 und 50 µm umfasst.

3. Dianhydrohexitol, verpackt nach einem der Ansprüche 1 oder 2, **dadurch gekennzeichnet, dass** das Dianhydrohexitol Isosorbid umfasst oder im Wesentlichen aus Isosorbid besteht.

4. Dianhydrohexitol, verpackt nach einem der vorhergehenden Ansprüche, **dadurch gekennzeichnet, dass** das thermoplastische Polymer der Schicht (A) und/oder der Schicht (B) aus Polyethylen, vorzugsweise aus verzweigtem Polyethylen, linearem Polyethylen und/oder Metallocen-Polyethylen besteht.

5. Dianhydrohexitol, verpackt nach einem der vorhergehenden Ansprüche, **dadurch gekennzeichnet, dass** das Dianhydrohexitol hermetisch verpackt ist, vorzugsweise unter einer Inertatmosphäre.

6. Dianhydrohexitol, verpackt nach einem der vorhergehenden Ansprüche, **dadurch gekennzeichnet, dass** das Antioxidationsmittel aus Kohleschwarz besteht.

7. Dianhydrohexitol, verpackt nach einem der vorhergehenden Ansprüche, **dadurch gekennzeichnet, dass** das Verpackungsmaterial eine Schicht (A) aus Polyethylen, beladen mit Kohleschwarz, und eine Schicht (B), die nicht mit Kohleschwarz beladen ist, in direktem Kontakt mit dem Dianhydrohexitol, umfasst.

8. Dianhydrohexitol, verpackt nach einem der vorhergehenden Ansprüche, **dadurch gekennzeichnet, dass** das Verpackungsmaterial weiterhin eine Barriereschicht umfasst, die wenig permeabel oder impermeabel für Wasserdampf und Sauerstoff sein kann (Schicht (c), angeordnet auf der Außenseite der Schicht (A).

9. Dianhydrohexitol, verpackt nach einem der vorhergehenden Ansprüche, **dadurch gekennzeichnet, dass** das Verpackungsmaterial auf Basis eines thermoplastischen Polymers eine Gesamtdicke von höchstens gleich 300 µm, vorzugsweise zwischen 30 und 250 µm hat.

10. Dianhydrohexitol, verpackt nach einem der vorhergehenden Ansprüche, **dadurch gekennzeichnet, dass** die Schicht (A) von 0,1 bis 10 Gew.-%, vorzugsweise von 0,2 bis 5 Gew.-% und insbesondere von 0,3 bis 3 Gew.-% Antioxidationsmittel enthält.

11. Verwendung eines Verpackungsmaterials auf Basis eines thermoplastischen Polymers zum Verpacken eines Dianhydrohexitols, vorzugsweise in fester Form, **gekennzeichnet dadurch, dass** das Verpackungsmaterial mindestens eine Schicht aus thermoplastischem Polymer umfasst, enthaltend mindestens 0,1 Gew.-%, vorzugsweise mindestens 0,2 Gew.-%, mindestens eines Antioxidationsmittels (Schicht (A)), wobei die Schicht (A) entweder direkt in Kontakt ist mit dem Dianhdrohexitol oder getrennt ist von diesem durch ein thermoplastisches Polymer (Schicht (B)) das eine Dicke von höchstens gleich 150 µm, vorzugsweise von höchstens gleich 120 µm aufweist, und dadurch, dass das thermoplastische Polymer der Schicht (A) und/oder der Schicht (B) unabhängig ausgewählt ist aus Polyethylen, Polypropylen, den Copolymeren von Ethylen und Propylen und den Gemischen von diesen.

12. Verfahren zum Verpacken von Dianhydrohexitol, vorzugsweise in fester Form, umfassend das Einbringen des Dianhydrohexitols in eine Verpackung aus einem Material auf Basis eines thermoplastischen Polymers, und das Verschließen der Verpackung, wobei das Verfahren **dadurch gekennzeichnet ist, dass** das Verpackungsmaterial mindestens eine Schicht aus thermoplastischem Polymer umfasst, enthaltend mindestens 0,1 Gew.-%, vorzugsweise mindestens 0,2 Gew.-%, mindestens eines Antioxidationsmittels (Schicht (A)), wobei die Schicht (A) entweder direkt in Kontakt ist mit dem Dianhdrohexitol oder getrennt ist von diesem durch ein thermoplastisches Polymer (Schicht (B)) das eine Dicke von höchstens gleich 150 µm, vorzugsweise von höchstens gleich 120 µm aufweist, und dadurch, dass das thermoplastische Polymer der Schicht (A) und/oder der Schicht (B) unabhängig ausgewählt ist aus Polyethylen, Polypropylen, den Copolymeren von Ethylen und Propylen und den Gemischen von diesen.

## Claims

1. A dianhydrohexitol, preferably in solid form, packaged in a packaging material based on a thermoplastic polymer, **characterized by** the fact that the packaging material comprises at least one layer of thermoplastic polymer containing at least 0.1% by weight, preferably at least 0.2% by weight, of at least one antioxidant (layer (A)), said layer (A) either being directly in contact with the dianhydrohexitol or being separated from the latter by a layer made from a thermoplastic polymer (layer (B)) having a thickness at most equal to 150 µm, preferably at most equal to 120 µm, and by the fact that the thermoplastic polymer of the layer (A) and/or of the layer (B) is chosen independently from polyethylene, polypropylene, copolymers of ethylene and of propylene, and mixtures thereof.

2. The packaged dianhydrohexitol as claimed in claim 1, **characterized by** the fact that the layer (B) has a thickness between 10 and 100 µm, preferably between 15 and 50 µm.

3. The packaged dianhydrohexitol as claimed in claim 1 or 2, **characterized by** the fact that the dianhydrohexitol comprises isosorbide or is essentially constituted of isosorbide.

4. The packaged dianhydrohexitol as claimed in any one of the preceding claims, **characterized by** the fact that the thermoplastic polymer of the layer (A) and/or of the layer (B) is polyethylene, preferably branched polyethylene, linear polyethylene and/or metallocene polyethylene.

5. The packaged dianhydrohexitol as claimed in any one of the preceding claims, **characterized by** the fact that the dianhydrohexitol is hermetically packaged, preferably under an inert atmosphere.

6. The packaged dianhydrohexitol as claimed in any one of the preceding claims, **characterized by** the fact that the antioxidant is carbon black.

7. The packaged dianhydrohexitol as claimed in any one of the preceding claims, **characterized by** the fact that the packaging material comprises a layer (A) made from polyethylene filled with carbon black and a layer (B) made from polyethylene that is not filled with carbon black, directly in contact with the dianhydrohexitol.

8. The packaged dianhydrohexitol as claimed in any one of the preceding claims, **characterized by** the fact that the packaging material also comprises a barrier layer that is not very permeable or that is impermeable to water vapor and to oxygen (layer (C)), located on the outside of the layer (A).

9. The packaged dianhydrohexitol as claimed in any one of the preceding claims, **characterized by** the fact that the packaging material based on a thermoplastic polymer has a total thickness at most equal to 300 µm, preferably between 30 and 250 µm.

10. The packaged dianhydrohexitol as claimed in any one of the preceding claims, **characterized by** the fact that the layer (A) contains from 0.1 to 10% by weight, preferably from 0.2 to 5% by weight and in particular from 0.3 to 3% by weight of antioxidant.

11. The use of a packaging material based on a thermoplastic polymer for packaging a dianhydrohexitol, preferably in solid form, **characterized by** the fact that the packaging material comprises at least one layer of thermoplastic polymer containing at least 0.1% by weight, preferably at least 0.2% by weight, of at least one antioxidant (layer (A)), said layer (A) either being directly in contact with the dianhydrohexitol or being separated from the latter by a layer made from a thermoplastic polymer (layer (B)) having a thickness at most equal to 150 µm, preferably at most equal to 120 µm, and by the fact that the thermoplastic polymer of the layer (A) and/or of the layer (B) is chosen independently from polyethylene, polypropylene, copolymers of ethylene and of propylene, and mixtures thereof.

12. A process for packaging dianhydrohexitol, preferably in solid form, comprising the introduction of said dianhydrohexitol into a packaging made from a material based on a thermoplastic polymer, and the sealing of said packaging, said process being **characterized by** the fact that the packaging material comprises at least one layer of thermoplastic polymer containing at least 0.1% by weight, preferably at least 0.2% by weight, of at least one antioxidant (layer (A)), said layer (A) either being directly in contact with the dianhydrohexitol or being separated from the latter by a layer made from a thermoplastic polymer (layer (B)) having a thickness at most equal to 150 µm, preferably at most equal to 120 µm, and by the fact that the thermoplastic polymer of the layer (A) and/or of the layer (B) is chosen independently from polyethylene, polypropylene, copolymers of ethylene and of propylene, and mixtures thereof.
